(19) Europäisches Patentamt

European Patent Office

Office européen des brevets

(11) **EP 4 738 367 A1**

(12) **EUROPEAN PATENT APPLICATION**

(43) Date of publication:
06.05.2026 Bulletin 2026/19

(21) Application number: 25211210.7

(22) Date of filing: **24.10.2025**

(51) International Patent Classification (IPC):
**G16B 15/00** (2019.01)     **G16B 15/20** (2019.01)
**G16B 40/20** (2019.01)

(52) Cooperative Patent Classification (CPC):
**G16B 40/20; G16B 15/00; G16B 15/20**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **04.11.2024 US 202463716140 P
26.06.2025 US 202519251485**

(71) Applicant: **Microsoft Technology Licensing, LLC
Redmond, WA 98052 (US)**

(72) Inventors:
  • **FOONG, YUE KWANG
    Redmond, 98052 (US)**
  • **JIMENEZ LUNA, JOSE SALVADOR
    Redmond, 98052 (US)**
  • **CLEGG, SARAH
    Redmond, 98052 (US)**
  • **ABDIN, OSAMA
    Redmond, 98052 (US)**

  • **GASTEGGER, MICHAEL
    Redmond, 98052 (US)**
  • **XIE, YU
    Redmond, 98052 (US)**
  • **HEMPEL, TIM
    Redmond, 98052 (US)**
  • **SATORRAS, VICTOR GARCÍA
    Redmond, 98052 (US)**
  • **VEELING, BASTIAAN SJOUKE
    Redmond, 98052 (US)**
  • **NOE, FRANK
    Redmond, 98052 (US)**
  • **SCHNEUING, ARNE
    Redmond, 98052 (US)**
  • **YANG, SOOJUNG
    Redmond, 98052 (US)**

(74) Representative: **CMS Cameron McKenna Nabarro
Olswang LLP
Cannon Place
78 Cannon Street
London EC4N 6AF (GB)**

(54) **PREDICTION OF PROTEIN STRUCTURE ENSEMBLES**

(57)     A computing system (10) for predicting protein structure ensembles includes processing circuitry (18) configured to, in a first training phase, ingest a synthetic dataset of protein sequences, perform structure-based clustering on the synthetic dataset to produce clusters of protein structures, filter the clusters of protein structures, and train a diffusion model (66) on training pairs. In a second training phase, the processing circuitry (18) receives a predicted protein structure for an input training protein sequence from the diffusion model (66), and compares the predicted protein structure to a corresponding training protein structure from a molecular dynamics simulation. In a third training phase, the processing circuitry (18) receives a predicted value for a property of sampled protein structures, compares the predicted value to an actual value of the property, and backpropagates the diffusion model (66) with the difference. The diffusion model (66) estimates a denoised protein structure corresponding to the given protein sequence in fifteen or fewer denoising steps.

**EP 4 738 367 A1**

**Description**

BACKGROUND

Biomolecules, such as proteins and ribonucleic acids (RNA), are fundamental to gene expression, cellular functions, and biological processes. The ability to predict and manipulate different three-dimensional (3D) structures that biomolecules adopt and switch between, and the affinity with which they bind to other molecules, is of fundamental importance for advancing biological research, as well as for pharmaceutical and biotechnology industries. However, many biomolecular mechanisms cannot be directly observed via laboratory experiments. While molecular dynamics (MD) simulations can be used for certain molecular property simulations, such as dynamics in the folded protein state, protein folding and conformational changes, and utilized for industrial applications such as drug discovery, such MD simulations require sampling a huge and complex conformational space, thereby resulting in either impractical computational costs or uncontrollable inaccuracies.

SUMMARY

To address the issues discussed herein, a computing system for predicting protein structure ensembles is provided. According to one aspect, a computing system includes processing circuitry configured to execute instructions using portions of associated memory to implement a protein structure ensemble prediction model. In a first training phase, the processing circuitry is configured to ingest a synthetic dataset of protein sequences, identify protein sequences in the synthetic data having structurally heterogeneous predictions, perform structure-based clustering on the protein sequences based on the structurally heterogeneous predictions, filter the clustered protein sequences to remove disordered sequences and clusters having a single representative, generate training pairs for a diffusion model included in the protein structure ensemble prediction model, and train the diffusion model on the training pairs. In a second training phase, the processing circuitry is configured to sample a training protein sequence and a corresponding training protein structure from a molecular dynamics simulation, corrupt the corresponding training protein structure, input the training protein sequence and the corrupted version of the corresponding training protein structure into the diffusion model, receive a predicted uncorrupted protein structure corresponding to the input training protein sequence from the diffusion model, and compare the predicted uncorrupted protein structure from the diffusion model to the uncorrupted corresponding training protein structure sampled from the molecular dynamics simulation. In a third training phase, the processing circuitry is configured to instruct the diffusion model to sample a plurality of structures for a given protein sequence, receive a predicted value for a property of a distribution of the plurality of sampled structures, compare the predicted value of the property from the diffusion model to an actual value of the property, calculate a difference between the predicted value of the property and the actual value of the property, and backpropagate the diffusion model with the calculated difference to minimize a loss function. The diffusion model is configured to estimate a denoised protein structure corresponding to the given protein sequence in fifteen or fewer denoising steps.

This Summary is provided to introduce a selection of concepts in a simplified form that are further described below in the Detailed Description. This Summary is not intended to identify key features or essential features of the claimed subject matter, nor is it intended to be used to limit the scope of the claimed subject matter. Furthermore, the claimed subject matter is not limited to implementations that solve any or all disadvantages noted in any part of this disclosure.

BRIEF DESCRIPTION OF THE DRAWINGS

FIG. 1 shows a schematic diagram of a computing system for predicting protein structure ensembles, according to one embodiment of the present disclosure.

FIGS. 2A and 2B show an inference phase for a protein structure ensemble prediction model in accordance with the computing system of FIG. 1.

FIGS. 3A to 3C show a training pipeline for a protein structure ensemble prediction model in accordance with the computing system of FIG. 1.

FIGS. 4A to 4C show a flowchart of a computerized method for training a model to predict protein structure ensembles, according to an example implementation of the present disclosure.

FIG. 5 shows an example computing environment according to which the embodiments of the present disclosure may be implemented.

DETAILED DESCRIPTION

Proteins and their complexes constitute the functional building blocks of life and are central to drug discovery and development. They are the workhorses in biotechnological processes such as gene editing, enzymatic catalysis, and the formation of biomaterials. Understanding how proteins work, and how their function is affected by introducing other molecules or changing their sequence, is therefore one of the grand challenges for science and technology.

Molecular biology is characterized by three pillars of understanding: sequence, structure, and function. Next-generation sequencing, which emerged from the human genome project, has made the determination of protein sequences routine. Experimentally determined three-dimensional (3D) structures have been deposited in the Protein Data Bank (PDB), and the emergence of deep learning protein folding models have leveraged the information contained in sequence databases and the PDB to predict many 3D protein structures on a large scale with near experimental accuracy.

In contrast to protein sequence and structure, the development of a scalable and accurate technology for determining the mechanistic basis of biomolecular function, and how biological processes and drug intervention work at a molecular level, remains a challenge. Single-molecule experiments can provide the time evolution and full equilibrium distributions of one or a few observables, such as an intramolecular distance. Cryo-electron microscopy (cryo-EM) can resolve multiple conformational states of biomolecular complexes along with their probabilities, Boltzmann generators can efficiently generate samples of 3D molecular structures from a defined equilibrium distribution, and denoising diffusion models have become widely used in protein structure prediction and design. However, the application of these techniques at scale has been prohibited by time, expense, accuracy, and/or technical challenges. The situation is similar for molecular dynamics (MD) simulation, which is, in principle, a universal tool to explore structure and dynamics of biomolecules at an all-atom resolution, but the sampling problem makes even simple operations, such as folding or association of small proteins, a feat of epic computational costs, even with dedicated supercomputers or enhanced sampling methods. Lacking a scalable tool, there currently exists a detailed mechanistic understanding of biomolecular function for only a few anecdotal cases.

Design Principles

In view of the issues discussed above, a computing system 10 for predicting protein structure ensembles is provided. Utilizing a protein structure ensemble prediction model 30, the computing system 10 has applicability to predict many 3D protein structures from a protein's equilibrium distribution at near-experimental accuracy at large scale. The model disclosed herein includes a generative model that takes a protein sequence as input and generates random samples from an approximated equilibrium distribution of structures for the protein sequence. The generative model includes a diffusion model that is pre-trained with protein sequences and ground truth structures from data sources such as public databases and/or specially constructed synthetic data, and fine-tuned on molecular dynamics (MD) simulations and experimental datapoints of protein thermodynamics, resulting in a highly scalable diffusion model that can generate thousands of statistically independent samples of biomolecular structures from the equilibrium distribution of that biomolecule for a given protein sequence in one graphics processing unit (GPU) hour.

The diffusion model emulates the distributions and energy landscapes of ultralong MD simulations orders of magnitude faster than all-atom and coarse-grained MD and with errors that are on the same order than the differences between different state-of-the-art all-atom forcefields. These features provide the model with the ability to predict conformational changes, emulate equilibrium distributions, and predict thermodynamic properties.

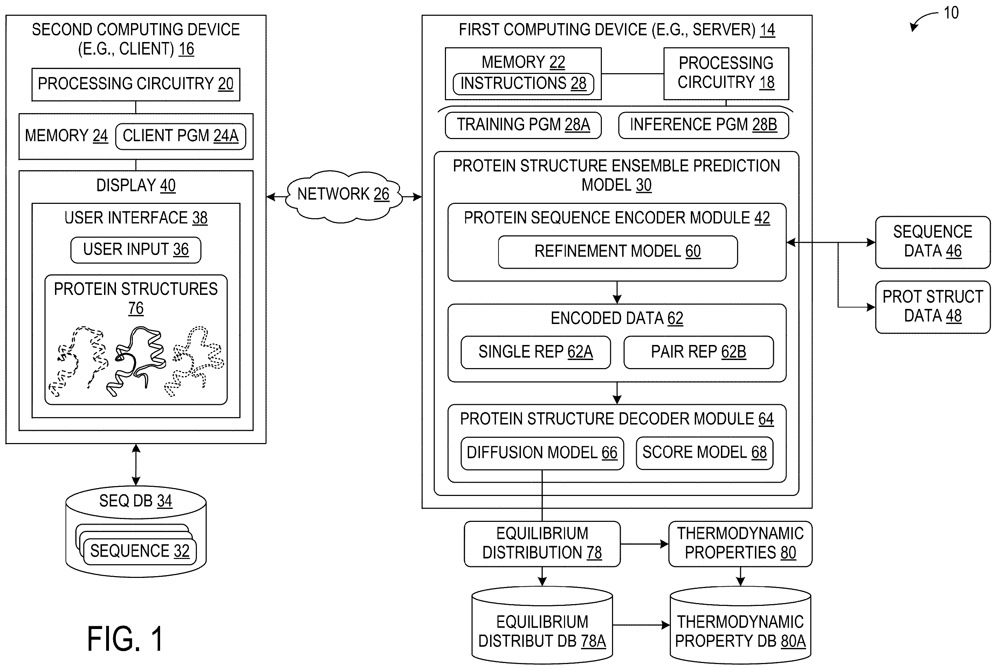

The following discussion provides an overview of the theoretical underpinnings and design principles that gave rise to the architecture of the protein structure ensemble prediction model 30, and how the model is trained. These sections are followed by a detailed description of example embodiments of systems and methods for a protein structure ensemble prediction model 30 during training and inference phases, with reference to Figs. 1-4.

Protein Sequence Encoder

A protein sequence, i.e., amino acid sequence, is input to the model and encoded via a protein sequence encoder to compute single and pair representations. This may be performed with a simplified version of AlphaFold 2 and pre-trained sequence representations, for example. Many-against-many sequence searching (MMseqs) interfaced with an accelerated structure prediction engine (e.g., Colabfold) with default parameters for efficient and large-scale multiple sequence alignment (MSA) search is used. Templates are completely excluded, and AlphaFold 2 recycling iterations are removed. During generation, the random seed is set to 0, and the single and pair embeddings are used. As the protein sequence encoder depends on no other variables than the protein sequence, the single and pair embeddings for all proteins used in training and inference may be pre-computed once and then stored for fast retrieval.

## Coarse-grained Protein Structure Representation

The protein structure ensemble prediction model generates 3D protein structures with a coarse-grained representation in which the backbone heavy atoms of the protein are represented via a backbone frame representation, with side-chains and hydrogen atoms not explicitly modeled. To convert an all-atom protein conformation to its backbone frame representation for a given residue, a respective $C_\alpha$ atom coordinate $r \in \mathbb{R}^3$ is used and the Gram-Schmidt algorithm on the displacement vectors $C_\alpha \to N$ and $C_\alpha \to C$ is performed. This yields an orthonormal basis which can be represented as a rotation matrix $Q \in SO(3)$. Repeating this for each residue, a sequence of position-orientation tuples, $x := \{(r_i, Q_i)\}_{i=1}^N$, is obtained for all N protein residues.

To recover the Cartesian backbone atom positions from the frame representation, a reference backbone heavy-atom frame per residue type with idealized atom positions is determined. For example, for the amino acid alanine ($C_3H_7NO_2$), the idealized frame atom positions are:

$$
\begin{array}{c}
N \\
C_\alpha \\
C \\
C_\beta \\
O
\end{array}
\begin{pmatrix}
-0.525 & 1.363 & 0.000 \\
0.000 & 0.000 & 0.000 \\
1.526 & 0.000 & 0.000 \\
-0.529 & -0.774 & -1.205 \\
0.627 & 1.062 & 0.000
\end{pmatrix}.
$$

Then, the rotation matrix $Q_n$ is applied to obtain the rotated frame, and the position vector $r_n$ is added to the coordinates of all the atoms in the frame. It will be appreciated that, since the $C_\alpha$ is at the origin of the idealized frame, it will be at exactly location $r_n$ upon applying this transformation.

## Diffusion Conditional Generative Model

The protein structure ensemble prediction model acts as a sequence-conditional generative model: given a protein amino acid sequence, the model parameterizes a distribution of backbone conformational states. Here, let $S = (a_1, a_2, \ldots, a_N)$ be a protein sequence with $N$ residues $a_i \in \mathcal{R}$ from the set of 20 standard amino acids. The protein structure ensemble prediction model includes a diffusion model that can be used to sample 3D protein conformations x from a conditional distribution (Equation 1).

$$
(1) \qquad\qquad x_0 \sim p_\theta(x|S),
$$

where θ are learnable weights that parameterize a neural network that acts as a score model $s_\theta(x|S)$. It will be appreciated that, as the dimensionality of x depends on the number of residues $N$, the dimensionality of the space over which the protein structure ensemble prediction model defines a distribution depends on the length of $S$. The sampling procedure that characterizes $p_\theta(x|S)$ is given by simulating the estimated inverse of a forward diffusion process, defined by a stochastic differential equation on the space of backbone frame representations x (Equation 2).

$$
(2) \qquad\qquad dx = f(x,t)dt + G(x,t)dw,
$$

where w is a standard Wiener process, and f and G, which are drift and diffusion coefficients, respectively, are functional hyperparameters. The drift and diffusion coefficients were chosen such that all residues, as well as their positions r and orientations Q, are corrupted independently. The positions are corrupted with a variance-preserving Stochastic Differential Equation (SDE) and a cosine noise schedule, with the marginal distribution of the change in orientation after time t being represented in Equation 3.

$$
(3) \qquad \mathcal{IG}_{SO(3)}(\omega, \sigma^2) = \frac{1-\cos(\omega)}{\pi} \sum_{l=0}^{\infty} (2l+1)\, e^{-l(l+1)\frac{\sigma^2}{2}} \frac{\sin\left(\left(l+\frac{1}{2}\right)\omega\right)}{\sin\left(\frac{\omega}{2}\right)},
$$

where $\omega$ is the angle between rotations $Q_t$ and $Q_0$, computed as Equation 4.

$$(4) \qquad \omega = \arccos\left(\text{trace}(Q_t^\top Q_0)/2 - 1/2\right).$$

To denote the probability distribution of x at diffusion time t when x is corrupted in the above way, $p(x, t)$ is used, with the boundary condition that $p(x, 0) = p(x)$, i.e., the target distribution. If the initial positions $r_0$ are bounded, then $p(x, 0)$ is close to a simple prior distribution under which positions have a standard isotropic Gaussian distribution, and orientations are uniformly distributed.

It has been shown that by training on samples x(0) from $p(x)$ together with corresponding samples from the conditional distribution of x(t) given x(0), a model can approximate the score $\nabla_x p(x, t)$. Furthermore, if the score is known, SDEs under which the evolution of the probability density $\frac{\partial}{\partial t} p(x, t)$ is reversed can be constructed. Starting by sampling positions r and orientations Q from the prior and gradually denoising by simulating one such SDE from $t = 0$ to $t = 1$, it is possible to approximately sample from the target distribution.

Score Model

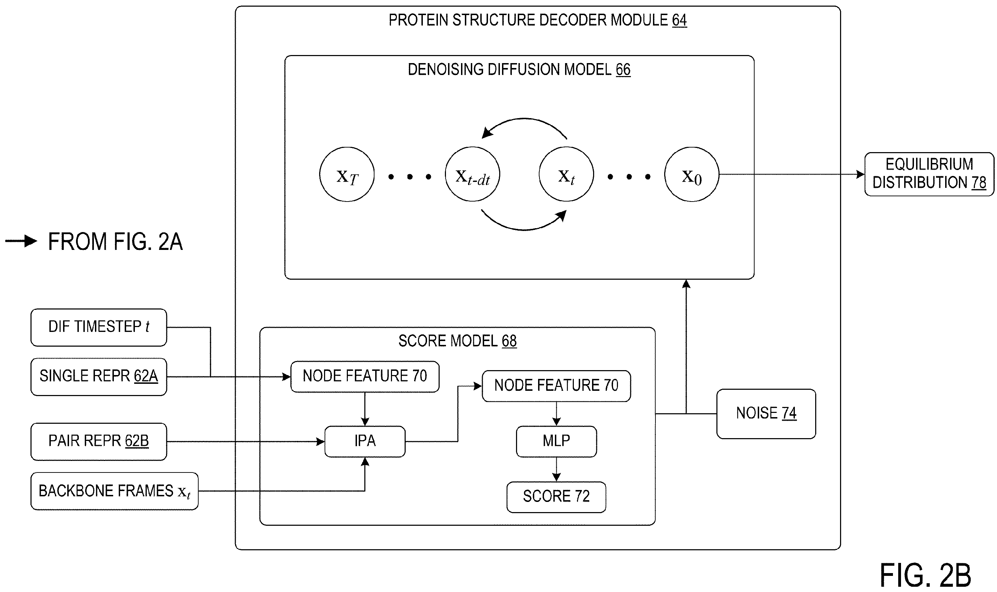

A score model receives single representations $h := \{h_i\}_{i=1}^N$ and pair representations of the protein sequence $z := \{z_{ij}\}_{i,j=1}^N$, corrupted frames $x := \{r_i, Q_i\}_{i=1}^N$, relative sequence positions $p := \{p_i\}_{i=1}^N$, and a diffusion timestep t, and predicts the score $s_\theta(x, h, z, t)$. The score model resembles the structure modules of the AlphaFold2 and Distributional Graphormer models, and uses invariant point attention (IPA) transformer and multilayer perceptron (MLP) feedforward architecture. As discussed below, Fig. 2B shows an overview of the architecture, and a detailed description is provided in Algorithm 1.

---

**Algorithm 1: Score model $s_\theta(x, h, z, t)$**

---

**Require:** single representations $h_i$, pair representations $z_{ij}$, positions $r_i$, rotations $Q_i$, timestep $t$, relative sequence positions $p_i$.

1: $h_i \longleftarrow \text{Linear}(\text{LayerNorm}(h_i)) + \text{Sinusoidal}(t)$

2: $z_{ij} \longleftarrow \text{LinearNoBias}(\text{LayerNorm}(z_{ij})) + \text{Embedding}(\text{Bucketize}(p_i))$

3: **for** layer=1, …, 8 **do**

4: $\quad \{h_i\}$ +=Dropout(IPA($\{\text{LayerNorm}(h_i)\}, \{z_{ij}\}, \{r_i\}, \{Q_i\}$))

5: $\quad h_i$ +=Dropout(Linear(Dropout(gelu(Linear(LayerNorm($h_i$))))))

6: **end for**

7: $s_r = \text{Linear}(\text{relu}(\text{Linear}(\text{LayerNorm}(h_i))))$

8: $s_Q = \text{Linear}(\text{relu}(\text{Linear}(\text{LayerNorm}(h_i))))$

9: **return** $s_r, s_Q$

---

The translation and rotation scores produced by the score model in Algorithm 1 are defined in the local coordinate frame of each residue, and are invariant under rotation or translation of the entire structure. During denoising, the updates to backbone atom positions are therefore equivariant under rotation and translation of the whole structure.

Pre-Training the Diffusion Model with Protein Sequences and Structures

It has been observed that proteins with similar sequences can have similar conformational landscapes, but changes in protein sequence or other perturbations, such as the binding of a small molecule, will change the relative probabilities of the accessible conformations. As such, in a first training phase, i.e., pre-training, the diffusion model 66 is trained to capture the diversity of structures that each protein sequence can adopt. The priority at this stage is coverage, not accuracy, and the model may generate structures that are quite different from the Boltzmann distribution, but with a high level of diversity.

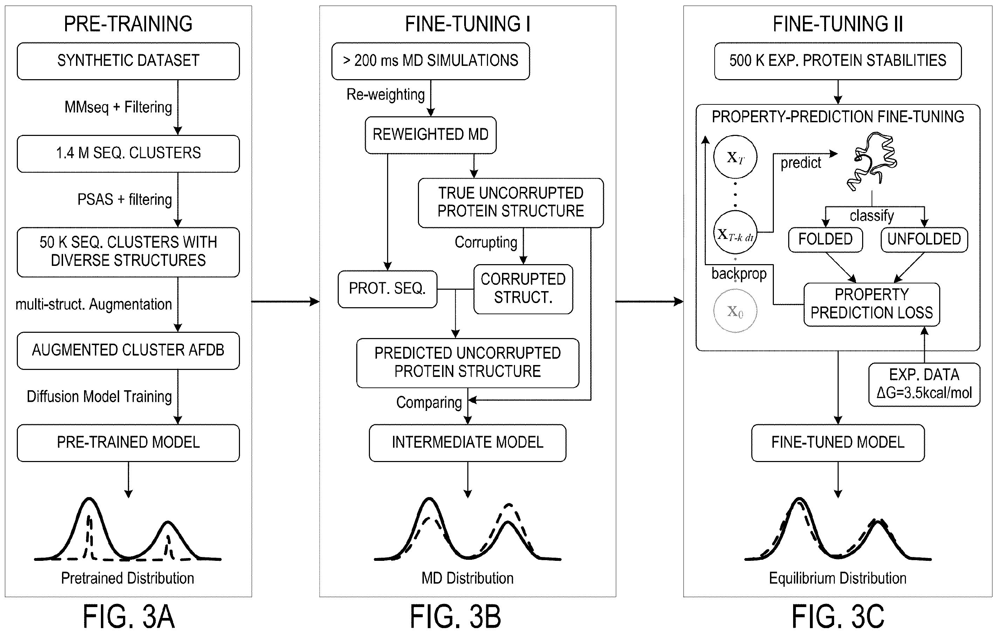

As described below with reference to Fig. 3A, a large synthetic dataset of highly flexible protein sequences may be derived from a protein structure database. A synthetic dataset of 200 million sequences is used as an example. One example database that can be used is the AlphaFold Protein Structure Database (AFDB). The protein structure database contains one or a small number of predicted structures for each of a wide variety of sequences. Starting with such a database, similar sequences with structurally heterogeneous predictions are identified via many-against-many sequence searching (MMseqs). An initial clustering of all sequences from the sequence database at 80% sequence identity and 70% coverage results in a set of more than 93 million sequence clusters. An additional clustering of the cluster centroids at 30% sequence identity yields approximately 1.4 million sequence clusters, each containing at least 10 members.

Within each set of sequences, structure-based clustering is performed using a protein structure alignment server (PSAS, e.g., Foldseek), with a sequence identity threshold of 70% at 90% coverage, and resulting clusters with only one representative are discarded. Clusters containing disordered representatives (i.e., being composed of more than 50% coil in their secondary structure) are additionally filtered out. For sequences in which structural heterogeneity is flagged due to missing regions in centroid proteins, structural alignments are performed in sequence-aligned regions of proteins, and cluster centroids with a template modeling (TM)-score greater than 0.9 to another centroid are filtered out. Finally, clusters lacking at least one structure having a predicted local distance different test (pLDDT) value greater than 80, and standard deviation less than 15 across residues, are removed.

After filtering, approximately fifty thousand (50K) sequence clusters with structural diversity remain in the training data set. The data is then augmented to artificially increase the variety of structures associated with each sequence. Training pairs for the diffusion model 66 are generated by randomly selecting a cluster and randomly selecting a structure from within the randomly selected cluster, and partnering the randomly selected structure with a sequence that corresponds to the highest pLDDT value structure from within the same cluster. The denoising score-matching loss is defined as a sum over residues, with the loss being set to zero for residues corresponding to insertions or deletions not present in the sequence having the highest pLDDT value. This training methodology encourages the model to sample diverse structures for each input sequence, and results in a pretrained diffusion model 66.

Fine-Tuning the Diffusion Model with Molecular Dynamics Simulations and Experimental Data

After the diffusion model 66 is pre-trained with the structure and sequence data, it is fine-tuned on MD simulations and experimental datapoints of protein thermodynamics in a second training phase and a third training phase. MD simulations model the movements of atoms in a protein over time, which results in a distribution of conformations of the protein that depend on the thermodynamic and kinetic properties of the protein.

For the MD simulations and experimental data portion of the fine-tuning training phase, two kinds of training steps are employed. In the second training phase (i.e., fine-tuning I), a protein sequence and a corresponding protein structure are sampled from an MD simulation and may be re-weighted as described in detail below. The corresponding protein structure is then corrupted, and the protein sequence and the corrupted version of the corresponding protein structure are input to the model. The diffusion model 66 predicts the uncorrupted protein structure, which is compared to the true uncorrupted protein structure initially sampled from the MD simulation.

In the third training phase (i.e., fine-tuning II), the diffusion model 66 is used to sample a plurality of structures for a protein sequence, and a property of the distribution of these sampled structures may be computed. The property may be, for example, the probability or free energy difference between different long-lived (metastable) states, including the free energy difference between folded and unfolded states, the distribution or the mean value of a distance between two amino acids in the three-dimensional structure of the protein, and/or the distribution and expectation values of secondary and tertiary structures of the protein. As such, the property may be a class, a value, or a tensor. Rather than cycling through hundreds of denoising steps, the diffusion model 66 is configured to estimate the final denoised protein structure after a small, predetermined number of denoising steps, e.g., in fifteen or fewer denoising steps. In some implementations, the diffusion model 66 can estimate the final denoised protein structure in ten or fewer denoising steps, and in other implementations in eight or fewer denoising steps.

From a set of one or more estimated denoised protein structures for the same sequence, the diffusion model 66 predicts a value or class for a property of the distribution of structures of that protein. The predicted value, class, or tensor from the diffusion model 66 is compared to experimental datapoints indicating one or more actual values or classes derived from laboratory experiments, and the difference between the two values or classes is calculated. The value of the difference is then used to backpropagate the diffusion model to minimize the loss function.

As such, the two kinds of training steps allow the diffusion model to be fine-tuned first on MD simulations data, which results in an intermediate model, and then on experimental data, which produces the final fine-tuned model. Specifically, the model predicts a distribution of values for an input sequence (i.e., a per-structure property) by sampling several structures for the input sequence. A loss function is then used to steer the mean of the distribution towards an experimentally determined value, but still has the freedom to sample various structures across the distribution. Thus, not every predicted structure will follow the mean. It will be appreciated that the diffusion model includes two loss terms: a score matching loss term, as typically used for training diffusion models, and a loss term that takes into account class probabilities.

Weighting the Training Data

Due to the prohibitive cost of MD simulations, most protein molecular dynamics simulations are limited in their simulation time and do not represent the Boltzmann distribution. Rather, they are biased towards the starting conditions of the simulations. To generate a representative ensemble of protein conformations from molecular dynamics (MD) simulation data that do not reach equilibrium within the simulation timeframe, a reweighting procedure is performed over protein structures in the molecular dynamics simulation to approximate an equilibrium distribution. Multiple orthogonal sources of information about the Boltzmann distribution are available and may be used for re-weighting the MD simulation data. For example, experimental protein stability measurements provide the free energy difference between the folded and the unfolded state of the protein, which is related to the probabilities of folded vs. unfolded states by the Boltzmann distribution. Additionally, Markov state models (MSMs) provide a set of tools that are commonly applied to MD simulations in order to estimate the Boltzmann distribution from simulation data which have not reached equilibrium yet. MSMs exploit the time information in the MD simulations and extract equilibrium weights by a spectral analysis of the transition matrix.

In both cases, each molecular conformation, i.e., protein structure, from an MD simulation can be assigned a probability weight. For re-weighting with experimental protein stability measurements, each frame in the simulation is classified as folded or unfolded using a geometric criterion and assigned a weight such that the weighted proportion of frames that are classified as folded is the same as the experimentally determined probability of being in a folded state. For re-weighting with MSM probabilities, an MSM is estimated from the molecular dynamics trajectories, and MSM equilibrium weights are assigned to a corresponding frame. During the second training phase, the corresponding training protein structure is sampled from the molecular dynamics simulation with a probability according to the re-weighted protein structures.

Example Embodiments

In accordance with principles discussed above, a specific example embodiment of protein structure ensemble prediction model according to the present disclosure will now be described, with reference to FIGS. 1-4.

Referring initially to FIG. 1, the computing system 10 includes at least one computing device. The computing system 10 is illustrated as having a first computing device 14 including processing circuitry 18 and memory 22, and a second computing device 16 including processing circuitry 20 and memory 24. The illustrated implementation is exemplary in nature, and other configurations are possible. In the description below, the first computing device will be described as a server 14, the second computing device will be described as a client computing device 16, the server 14 and the client computing device 16 are in communication via a network 26, and respective functions carried out at each computing device 14, 16 will be described. It will be appreciated that in other configurations, the computing system 10 may include a single computing device that carries out the salient functions of both the first computing device 14 and second client computing device 16, and that the first computing device 14 could be a computing device other than server. In other alternative configurations, functions described as being carried out at the first computing device 14 may alternatively be carried out at the second computing device 16 and vice versa. The first computing device 14 will be described in the example embodiment of FIG. 1 as a server 14 and the second computing device 16 as a client computing device 16.

Continuing with FIG. 1, the processing circuitry 18 is configured to execute instructions 28 using portions of associated memory 22 to implement a protein structure ensemble prediction model 30 hosted at the server 14. The instructions 28 include a training program 28A, which, when executed by the processing circuitry 18, implements a training algorithm in a training phase to train the protein structure ensemble prediction model 30, and an inference program 28B, which when executed by the processing circuitry 18 causes the trained model to perform inference in an inference phase. An application programming interface (API) may be provided for communicating with the training program 28A and the inference program 28B, for example to/from second computing device 16.

At a high level, the protein structure ensemble prediction model 30 is trained, during the training phases discussed above, to process input during the inference phase of a nucleic acid or amino acid sequence 32 to thereby output a prediction of biomolecular properties of the associated protein based on domain similarities, molecular dynamics, and experimental datapoints over a range of predetermined time steps in the diffusion process. The sequence 32 may be stored in a sequence database 34, and entered as user input 36 via a user interface 38 of a client program 24A, which is displayed on a display 40 and/or included in the client computing device 16.

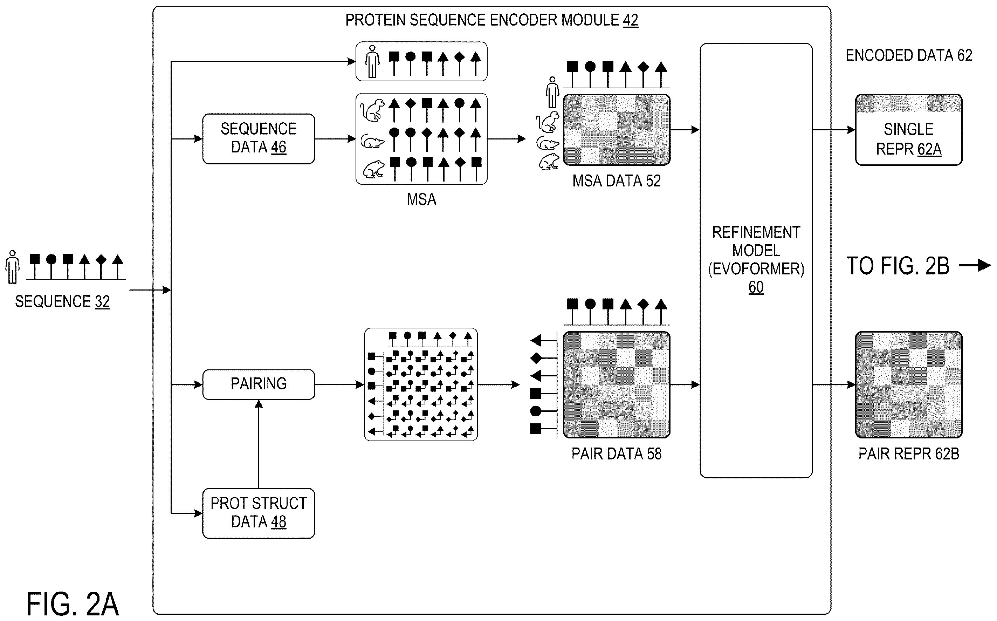

The protein structure ensemble prediction model 30 includes a protein sequence encoder module 42. As shown in detail in FIG. 2A, in the inference phase, the protein sequence encoder module 42 is configured to ingest the sequence 32 and perform searches for sequence data 46 and protein structure data 48 based on the sequence 32. The sequence data 46 may be one or more sequences of nucleic acid (i.e., an RNA or DNA sequence) or amino acids (i.e., a protein sequence). Multiple sequence alignments (MSAs) may be identified via a many-against-many sequence search, such as MMseqs2. The resulting alignment may be expressed as graph-structured data. An example representation of MSA data 52 is shown in FIG. 2A. It will be appreciated that the sequences may be derived from a variety of genomes, such as human, simian, murine, amphibian, and avian, for example. The sequence 32 and candidate protein structure data 48 may be paired and expressed as graph-structured data. An example representation of pair data 58 is shown in FIG. 2A.

The MSA data 52 and pair data 58 are passed to a refinement model 60 for further refinement. One suitable refinement model 60 is the Evoformer model, which is based on the transformer architecture. The refinement model 60 is configured to receive the MSA data 52 and pair data 58 as input, refine the representations of the MSA data 52 and the pair data 58, and output a joint latent (feature) representation as encoded data 62, including single representations 62A corresponding to the MSA data 52 and pair representations 62B corresponding to the pair data 58. The encoded data 62 is then fed to a denoising diffusion model 66 (discussed below) to predict molecular properties and structural features of the input sequence 32. As the protein sequence encoder module 42 depends on no variables other than the protein sequence 32, single and pair embeddings for all proteins that are generated by the refinement model 60 and used in training and inference are precomputed only once and stored for fast retrieval.

Continuing from FIG. 2A to FIG. 2B, with reference to FIG. 1, the encoded data 62 output from the protein sequence encoder module 42 is input to a protein structure decoder module 64. The protein structure decoder module 64 includes the denoising diffusion model 66 and a score model 68. The score model 68 is configured to receive the single representations 62A and pair representations 62B, corrupted frames, relative sequence positions, and a diffusion timestep, and predict the score $s_\theta(x, h, z, t)$, where s is the score, $\theta$ are learnable weights, h are single representations, z are pair representations, x are corrupted frames, and t is a diffusion timestep, as discussed above.

In some embodiments, node features 70, such as atom type, electronegativity, and hybridization state, may be included in the score. As described above, the score model 68 uses IPL and MLP architecture to determine the score 72 for the single representations 62A and pair representations 62B. The score 72 and noise 74 are input to the denoising diffusion model 66.

The denoising diffusion model 66 performs a reverse diffusion process, over a plurality of timesteps $t$, with $T$ indicating a total number of reverse diffusion timesteps t. When fine-tuning the diffusion model, the denoising diffusion model 66 is configured to estimate the final denoised ("clean") protein structure $x_0$ at an early stage in the denoising process, e.g., after a threshold number of denoising steps, thereby decreasing the time needed for the diffusion process, as well as vastly reducing memory requirements for training with regard to computing and storing the gradient of a loss function during backpropagation. In one example, T=35 with higher order sampler and the threshold number of steps is in a range of five to ten steps of denoising, which represents 14-28% of the entire denoising pipeline of 35 steps. In another example, the threshold number of denoising steps is twenty steps. This diffusion process can be referred to as an accelerated diffusion process since the entire diffusion pipeline is not computed, speeding up the computations and reducing memory requirements.

At the conclusion of the diffusion process, one or more predicted protein properties and/or structures are output from the protein structure decoder module 64. As indicated in FIG. 1, the protein structure ensemble prediction model 30 is configured to predict and output an equilibrium distribution 78, from which thermodynamic properties 80, such as protein stabilities in terms of folding free energy, can be predicted. The equilibrium distributions 78 and thermodynamic properties 80 may be stored in equilibrium distribution databases and thermodynamic property databases 78A, 80A, respectively. The equilibrium distribution 78 (i.e., ensemble of protein structures) may be displayed as protein structures 76 in the user interface 38. The protein structures 76 may show differences in the protein structure, such as domain motion, local unfolding, and binding pocket exposure or formation.

FIGS. 3A to 3C shows a training pipeline for the denoising diffusion model 66 included in the protein structure ensemble prediction model 30. Beginning with FIG. 3A, pre-training the denoising diffusion model 66 with structure and sequence clusters in the first training phase, as described above with reference to the pre-training section, enables the denoising diffusion model 66 to predict distinct structures based on protein sequences. Also as described above and shown in FIG. 3B, in the second training phase, a first fine-tuning step of the denoising diffusion model 66 is performed by training with MD simulations to generate an intermediate model. FIG. 3C shows the third training, which is the second fine-tuning step of backpropagating in view of experimental data to generate a fine-tuned model. With MD simulation data, the denoising diffusion model 66 can predict MD distributions, but lacks the ability to generate equilibrium distributions within acceptable limits of statistical accuracy. However, the denoising diffusion model 66 can be further fine-tuned by computing a class or value from the clean protein structure $x_0$, calculating a difference between the class or value from the denoising diffusion model 66 and a class or value derived from experimental data, such as thermodynamic values acquired during laboratory projects. The difference between the value predicted by the diffusion model and the experimental value is used to backpropagate the denoising diffusion model 66. Upon fine-tuning with MD simulations and experimental data, the diffusion model 66 can efficiently and accurately predict an equilibrium distribution for a given protein sequence, as indicated in FIG. 3C.

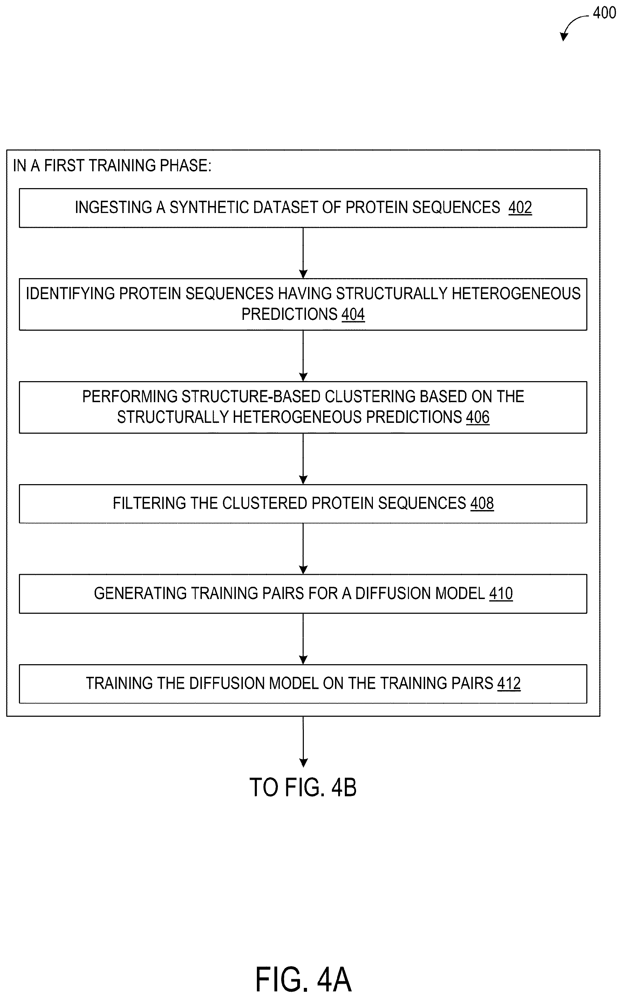

FIGS. 4A to 4C show a flowchart of a method 400 for training a model to predict protein structure ensembles. Method 400 may be implemented by the hardware and software of computing system 10 described above, or by other suitable hardware and software. Beginning with FIG. 4A, at step 402, the method 400 may include, in a first training phase, ingesting a synthetic dataset of protein sequences. The synthetic dataset may be derived from a protein structure database that contains highly flexible protein sequences, such as the AlphaFold Protein Structure Database (AFDB)

Proceeding from step 402 to step 404, at step 404 the method 400 may further include identifying protein sequences in the synthetic data having structurally heterogeneous predictions. As discussed above, the protein sequences having structurally heterogeneous predictions may be identified via many-against-many sequence searching.

Advancing from step 404 to step 406, at step 406 the method 400 may further include performing structure-based clustering on the protein sequences based on the structurally heterogeneous predictions. As discussed above, the structure-based clustering may be performed using a protein structure alignment server.

Continuing from step 406 to step 408, at step 408 the method 400 may further include filtering the clustered protein sequences to remove disordered sequences and clusters having a single representative. Disordered representatives may be defined as proteins being composed of more than 50% coil in their secondary structure, for example.

Proceeding from step 408 to step 410, at step 410 the method 400 may further include generating training pairs for a diffusion model included in the protein structure ensemble prediction model. As discussed above, generating the training pairs for the diffusion model may be achieved by randomly selecting a protein structure from a randomly selected structure-based cluster of protein sequences, and pairing the randomly selected protein structure with a protein sequence that corresponds to a highest predicted local distance different test value from within the randomly selected cluster.

Advancing from step 410 to step 412, at step 412, the method 400 may further include training the diffusion model on the training pairs.

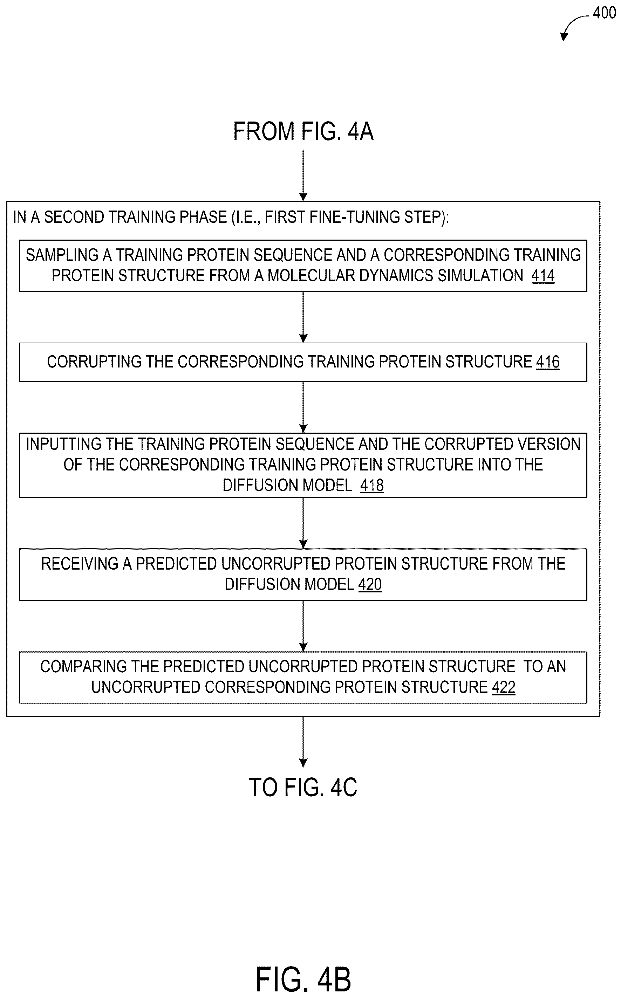

Turning to FIG. 4B, continuing from step 412 to step 414, at step 414 the method 400 may further include, in a second training phase, i.e., a first fine-tuning step, sampling a training protein sequence and a corresponding training protein structure from a molecular dynamics simulation.

Proceeding from step 414 to step 416, at step 416 the method 400 may further include corrupting the corresponding training protein structure.

Advancing from step 416 to step 418, at step 418 the method 400 may further include inputting the training protein sequence and the corrupted version of the corresponding training protein structure from the molecular dynamics simulation into the diffusion model. When the molecular dynamics simulation does not reach equilibrium within a simulation timeframe, a re-weighting procedure is performed over protein structures in the molecular dynamics simulation to approximate an equilibrium distribution, and the corresponding training protein structure is sampled from the molecular dynamics simulation with a probability according to the re-weighted protein structures. As described above, the re-weighting procedure may use Markov state model tools to estimate an equilibrium distribution over the conformational states. Alternatively, experimental data providing the relative proportions of folded and unfolded protein states is used to assign weights to the simulation-derived protein structures, such that the resulting ensemble reflects the experimentally observed equilibrium distribution.

Continuing from step 418 to step 420, at step 420 the method 400 may further include receiving, from the diffusion model, a predicted uncorrupted protein structure corresponding to the input training protein sequence.

Proceeding from step 420 to step 422, at step 422 the method 400 may further include comparing the predicted uncorrupted protein structure from the diffusion model to the uncorrupted corresponding protein structure sampled from the molecular dynamics simulation.

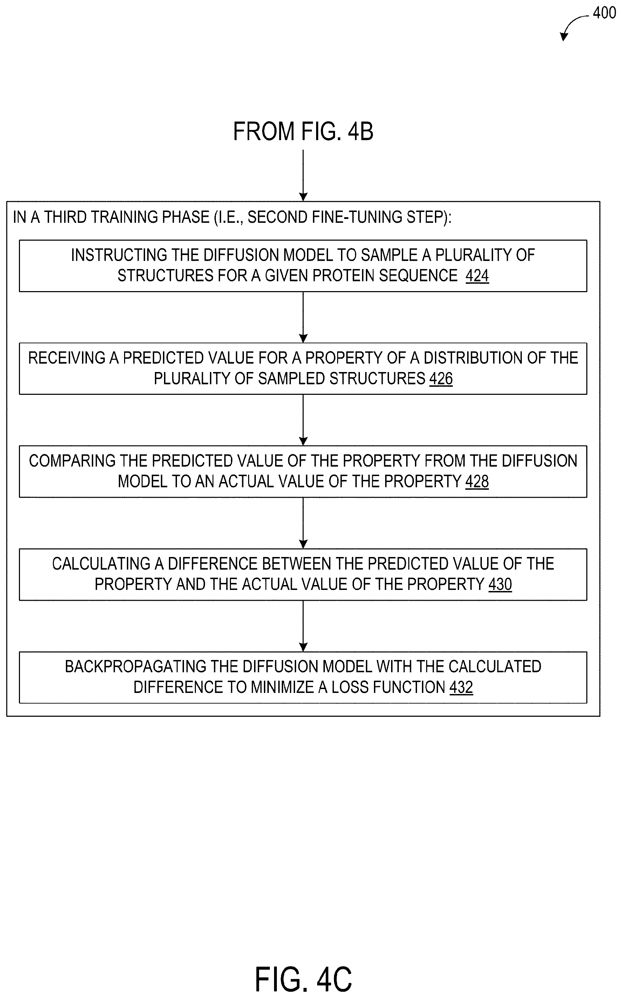

Turning to FIG. 4C, Advancing from step 422 to step 424, at step 424 the method 400 may further include, in a third training phase, i.e., a second fine-tuning step, instructing the diffusion model to sample a plurality of structures for a given protein sequence.

Continuing from step 424 to step 426, at step 426 the method 400 may further include receiving, from the diffusion model, a predicted value for a property of a distribution of the plurality of sampled structures. As discussed above, the property of the distribution of the plurality of sampled structures may be one of a class, a value, and a tensor. Additionally or alternatively, the property may be a value of a free energy difference between different metastable states or a mean value of a distance between two amino acids in a three-dimensional structure of the protein structure. The value of the free energy difference may include a value of a free energy difference between folded and unfolded states of the protein structure.

Proceeding from step 426 to step 428, at step 428 the method 400 may further include comparing the predicted value of the property from the diffusion model to an actual value of the property.

Advancing from step 428 to step 430, at step 430 the method 400 may further include calculating a difference between the predicted value of the property and the actual value of the property.

Continuing from step 430 to step 432, at step 432 the method 400 may further include backpropagating the diffusion model with the calculated difference to minimize a loss function. As discussed above, the diffusion model is configured to estimate a denoised protein structure corresponding to the given protein sequence in a small, predetermined number of denoising steps, such as fifteen or fewer denoising steps. In some implementations, the diffusion model estimates the denoised protein structure in as few as ten denoising steps, and in other implementations in as few as eight denoising steps.

The protein structure ensemble prediction model described herein provides a system for rapid emulation of key biomolecular properties, thus effecting efficient computational design in fields such as biomedical research, pharmaceutical engineering, and biotechnology. The model approximately emulates the distributions of protein structures that can be simulated by MD, but at a vastly lower inference cost that is reduced by three to six orders of magnitude. Additionally, the model can generate 3D protein structures from approximately the equilibrium distribution, making it a powerful tool for understanding protein functionality at the molecular level. Across all biomolecular modalities, the model has the potential to predict protein structure ensembles with free energy errors of less than 1 kcal/mol within less than one GPU hour and at a cost of less than one U.S. dollar per computational experiment. By leveraging the advantages of both diffusion models and experimental observations, and being customizable for molecular/protein ensemble sampling across various classes of molecules and experimental measurements with accurate and realistic results, the protein structure ensemble prediction model disclosed herein has the potential to enable significant advancements in protein design, drug discovery, and biophysics in academia, biotechnology industries, and beyond.

In some embodiments, the methods and processes described herein may be tied to a computing system of one or more computing devices. In particular, such methods and processes may be implemented as a computer-application program or service, an application-programming interface (API), a library, and/or other computer-program products.

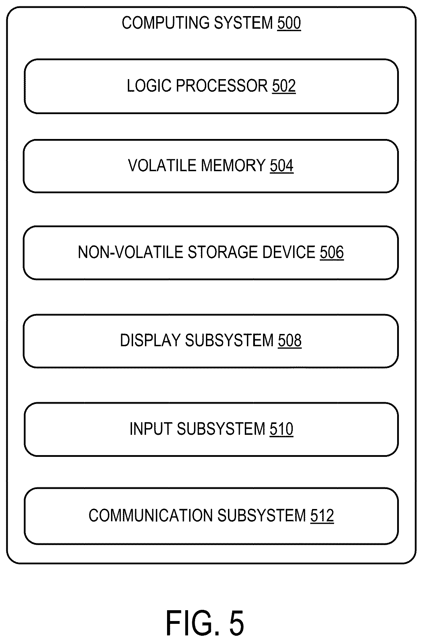

FIG. 5 schematically shows a non-limiting embodiment of a computing system 500 that can enact one or more of the methods and processes described above. Computing system 500 is shown in simplified form. Computing system 500 may embody the computer device 10 described above and illustrated in FIG. 1. Computing system 500 may take the form of one or more personal computers, server computers, tablet computers, home-entertainment computers, network computing devices, gaming devices, mobile computing devices, mobile communication devices (e.g., smart phone), and/or other computing devices, and wearable computing devices such as smart wristwatches and head mounted augmented reality devices.

Computing system 500 includes a logic processor, 502 volatile memory 504, and a non-volatile storage device 506. Computing system 500 may optionally include a display subsystem 508, input subsystem 510, communication subsystem 512, and/or other components not shown in FIG. 1.

Logic processor 502 includes one or more physical devices configured to execute instructions. For example, the logic processor may be configured to execute instructions that are part of one or more applications, programs, routines, libraries, objects, components, data structures, or other logical constructs. Such instructions may be implemented to perform a task, implement a data type, transform the state of one or more components, achieve a technical effect, or otherwise arrive at a desired result.

The logic processor may include one or more physical processors (hardware) configured to execute software instructions. Additionally or alternatively, the logic processor may include one or more hardware logic circuits or firmware devices configured to execute hardware-implemented logic or firmware instructions. Processors of the logic processor 502 may be single-core or multi-core, and the instructions executed thereon may be configured for sequential, parallel, and/or distributed processing. Individual components of the logic processor optionally may be distributed among two or more separate devices, which may be remotely located and/or configured for coordinated processing. Aspects of the logic processor may be virtualized and executed by remotely accessible, networked computing devices configured in a cloud-computing configuration. In such a case, these virtualized aspects are run on different physical logic processors of various different machines, it will be understood.

Non-volatile storage device 506 includes one or more physical devices configured to hold instructions executable by the logic processors to implement the methods and processes described herein. When such methods and processes are implemented, the state of non-volatile storage device 506 may be transformed-e.g., to hold different data.

Non-volatile storage device 506 may include physical devices that are removable and/or built-in. Non-volatile storage device 506 may include optical memory (e.g., CD, DVD, HD-DVD, etc.), semiconductor memory (e.g., ROM, EPROM, EEPROM, FLASH memory, etc.), and/or magnetic memory (e.g., hard-disk drive, floppy-disk drive, tape drive, MRAM, etc.), or other mass storage device technology. Non-volatile storage device 506 may include nonvolatile, dynamic, static, read/write, read-only, sequential-access, location-addressable, file-addressable, and/or content-addressable devices. It will be appreciated that non-volatile storage device 506 is configured to hold instructions even when power is cut to the non-volatile storage device 506.

Volatile memory 504 may include physical devices that include random access memory. Volatile memory 504 is typically utilized by logic processor 502 to temporarily store information during processing of software instructions. It will be appreciated that volatile memory 504 typically does not continue to store instructions when power is cut to the volatile memory 504.

Aspects of logic processor 502, volatile memory 504, and non-volatile storage device 506 may be integrated together into one or more hardware-logic components. Such hardware-logic components may include field-programmable gate arrays (FPGAs), program- and application-specific integrated circuits (PASIC / ASICs), program- and application-specific standard products (PSSP / ASSPs), system-on-a-chip (SOC), and complex programmable logic devices (CPLDs), for example.

The terms "module," "program," and "engine" may be used to describe an aspect of computing system 500 typically implemented in software by a processor to perform a particular function using portions of volatile memory, which function involves transformative processing that specially configures the processor to perform the function. Thus, a module, program, or engine may be instantiated via logic processor 502 executing instructions held by non-volatile storage device 506, using portions of volatile memory 504. It will be understood that different modules, programs, and/or engines may be instantiated from the same application, service, code block, object, library, routine, API, function, etc. Likewise, the same module, program, and/or engine may be instantiated by different applications, services, code blocks, objects, routines, APIs, functions, etc. The terms "module," "program," and "engine" may encompass individual or groups of executable files, data files, libraries, drivers, scripts, database records, etc.

When included, display subsystem 508 may be used to present a visual representation of data held by non-volatile storage device 506. The visual representation may take the form of a graphical user interface (GUI). As the herein described methods and processes change the data held by the non-volatile storage device, and thus transform the state of the non-volatile storage device, the state of display subsystem 508 may likewise be transformed to visually represent changes in the underlying data. Display subsystem 508 may include one or more display devices utilizing virtually any type of technology. Such display devices may be combined with logic processor 502, volatile memory 504, and/or non-volatile storage device 506 in a shared enclosure, or such display devices may be peripheral display devices.

When included, input subsystem 510 may comprise or interface with one or more user-input devices such as a keyboard, mouse, touch screen, or game controller. In some embodiments, the input subsystem may comprise or interface with selected natural user input (NUI) componentry. Such componentry may be integrated or peripheral, and the transduction and/or processing of input actions may be handled on- or off-board. Example NUI componentry may include a microphone for speech and/or voice recognition; an infrared, color, stereoscopic, and/or depth camera for machine vision and/or gesture recognition; a head tracker, eye tracker, accelerometer, and/or gyroscope for motion detection and/or intent recognition; as well as electric-field sensing componentry for assessing brain activity; and/or any other suitable sensor.

When included, communication subsystem 512 may be configured to communicatively couple various computing devices described herein with each other, and with other devices. Communication subsystem 512 may include wired and/or wireless communication devices compatible with one or more different communication protocols. As non-limiting examples, the communication subsystem may be configured for communication via a wireless telephone network, or a wired or wireless local- or wide-area network, such as a HDMI over Wi-Fi connection. In some embodiments, the communication subsystem may allow computing system 500 to send and/or receive messages to and/or from other devices via a network such as the Internet.

The following paragraphs provide additional support for the claims of the subject application. One aspect provides a computing system for predicting protein structure ensembles. The computing system comprises a computing device including processing circuitry configured to execute instructions using portions of associated memory to implement a protein structure ensemble prediction model. In a first training phase, the processing circuitry is configured to ingest a synthetic dataset of protein sequences, identify protein sequences in the synthetic dataset having structurally heterogeneous predictions, perform structure-based clustering on the identified protein sequences based on the structurally heterogeneous predictions to produce clusters of predicted protein structures, filter the clusters of predicted protein structures to remove disordered predicted protein structures and clusters comprising a single predicted protein structure, generate training pairs for a diffusion model included in the protein structure ensemble prediction model, and train the diffusion model on the training pairs. In a second training phase, the processing circuitry is configured to sample a training protein sequence and a corresponding training protein structure from a molecular dynamics simulation, corrupt the corresponding training protein structure, input the training protein sequence and the corrupted version of the corresponding training protein structure into the diffusion model, receive, from the diffusion model, a predicted uncorrupted protein structure corresponding to the input training protein sequence, and compare the predicted uncorrupted protein structure from the diffusion model to the corresponding training protein structure sampled from the molecular dynamics simulation. In a third training phase, the processing circuitry is configured to instruct the diffusion model to sample a plurality of structures for a given protein sequence, receive, from the diffusion model, a predicted value for a property of a distribution of the plurality of sampled structures, compare the predicted value of the property from the diffusion model to an actual value of the property, calculate a difference between the predicted value of the property and the actual value of the property, and backpropagate the diffusion model with the calculated difference to minimize a loss function. The diffusion model is configured to estimate a denoised protein structure corresponding to the given protein sequence in fifteen or fewer denoising steps.

In this aspect, additionally or alternatively, the protein sequences having structurally heterogeneous predictions are identified via many-against-many sequence searching.

In this aspect, additionally or alternatively, the structure-based clustering is performed using a protein structure alignment server.

In this aspect, additionally or alternatively, to generate the training pairs for the diffusion model, the processing circuitry is configured to randomly select a predicted protein structure from a randomly selected cluster of predicted protein structures, and pair the randomly selected predicted protein structure with a protein sequence that corresponds to a predicted protein structure having a highest predicted local distance different test value from within the randomly selected cluster.

In this aspect, additionally or alternatively, the property of the distribution of the plurality of sampled structures is one of a class, a value, and a tensor.

In this aspect, additionally or alternatively, the property is a value of a free energy difference between different metastable states or a mean value of a distance between two amino acids in a three-dimensional structure of the protein structure.

In this aspect, additionally or alternatively, the value of the free energy difference includes a value of a free energy difference between folded and unfolded states of the protein structure.

In this aspect, additionally or alternatively, when the molecular dynamics simulation does not reach equilibrium within a simulation timeframe, a re-weighting procedure is performed over protein structures in the molecular dynamics simulation to approximate an equilibrium distribution, and during the second training phase, the corresponding training protein structure is sampled from the molecular dynamics simulation with a probability according to the re-weighted protein structures.

Another aspect provides a computerized method for training a model to predict protein structure ensembles. The method utilizes processing circuitry and memory of one or more computing devices. In a first training phase, the method comprises ingesting a synthetic dataset of protein sequences, identifying protein sequences in the synthetic dataset having structurally heterogeneous predictions, performing structure-based clustering on the identified protein sequences based on the structurally heterogeneous predictions to produce clusters of predicted protein structures, filtering the clusters of predicted protein structures to remove disordered predicted protein structures and clusters comprising a single predicted protein structure, generating training pairs for a diffusion model included in the protein structure ensemble prediction model, and training the diffusion model on the training pairs. In a second training phase, the method comprises sampling a training protein sequence and a corresponding training protein structure from a molecular dynamics simulation, corrupting the corresponding training protein structure, inputting the training protein sequence and the corrupted version of the corresponding training protein structure into the diffusion model, receiving, from the diffusion model, a predicted uncorrupted protein structure corresponding to the input training protein sequence, and comparing the predicted uncorrupted protein structure from the diffusion model to the corresponding training protein structure sampled from the molecular dynamics simulation. In a third training phase, the method comprises instructing the diffusion model to sample a plurality of structures for a given protein sequence, receiving, from the diffusion model, a predicted value for a property of a distribution of the plurality of sampled structures, comparing the predicted value of the property from the diffusion model to an actual value of the property, calculating a difference between the predicted value of the property and the actual value of the property, and backpropagating the diffusion model with the calculated difference to minimize a loss function, wherein the diffusion model is configured to estimate a denoised protein structure corresponding to the given protein sequence in fifteen or fewer denoising steps.

In this aspect, additionally or alternatively, the method further comprises identifying the protein sequences having structurally heterogeneous predictions via many-against-many sequence searching.

In this aspect, additionally or alternatively, the method further comprises performing the structure-based clustering with a protein structure alignment server.

In this aspect, additionally or alternatively, the method further comprises generating the training pairs for the diffusion model by randomly selecting a predicted protein structure from a randomly selected cluster of predicted protein structures, and pairing the randomly selected predicted protein structure with a protein sequence that corresponds to a predicted protein structure having a highest predicted local distance different test value from within the randomly selected cluster.

In this aspect, additionally or alternatively, the property of the distribution of the plurality of sampled structures is one of a class, a value, and a tensor.

In this aspect, additionally or alternatively, the property is a value of a free energy difference between different metastable states or a mean value of a distance between two amino acids in a three-dimensional structure of the protein structure.

In this aspect, additionally or alternatively, the value of the free energy difference includes a value of a free energy difference between folded and unfolded states of the protein structure.

In this aspect, additionally or alternatively, the method further comprises, when the molecular dynamics simulation does not reach equilibrium within a simulation timeframe, performing a re-weighting procedure over protein structures in the molecular dynamics simulation to approximate an equilibrium distribution, and, during the second training phase, sampling the corresponding training protein structure from the molecular dynamics simulation with a probability according to the re-weighted protein structures.

Another aspect provides a computing system for predicting protein structure ensembles. The computing system comprises a computing device including processing circuitry configured to execute instructions using portions of associated memory to implement a protein structure ensemble prediction model. In an inference phase, the processing circuitry is configured to receive an input protein sequence, perform a search for protein sequence data based on the input protein sequence, identify and retrieve a subset of the protein sequence data having similarity to the input protein sequence, perform a multiple sequence alignment between the input protein sequence and the subset of the protein sequence data to produce multiple sequence alignment data, encode data from the multiple sequence alignment, the encoded data including single representations corresponding to the multiple sequence alignment data, and input the encoded data into a denoising diffusion model to predict molecular properties and structural features of the input protein sequence.

In this aspect, additionally or alternatively, the processing circuitry is further configured to perform a search for protein structure data based on the input protein sequence, identify and retrieve candidate protein structure data for candidates having a sequence-structure relationship with the input protein sequence, pair the input protein sequence and candidate protein structure data to produce pair data, and encode data from the pairing of the input protein sequence and the candidate protein structure data, the encoded data including pair representations corresponding to the pair data.

In this aspect, additionally or alternatively, the multiple sequence alignment data and the pair data from the pairing of the input sequence and the candidate protein structure data are input to a refinement model, and the refinement model outputs a joint latent representation as encoded data, the encoded data including the single representations corresponding to the multiple sequence alignment data and the pair representations corresponding to the pair data.

In this aspect, additionally or alternatively, the multiple sequence alignment between the input protein sequence and the subset of the protein sequence data is expressed as graph-structured data.

Another aspect provides a computing system for predicting protein structure ensembles includes processing circuitry configured to, in a first training phase, ingest a synthetic dataset of protein sequences, perform structure-based clustering on the synthetic dataset to produce clusters of protein structures, filter the clusters of protein structures, and train a diffusion model on training pairs. In a second training phase, the processing circuitry receives a predicted protein structure for an input training protein sequence from the diffusion model, and compares the predicted protein structure to a corresponding training protein structure from a molecular dynamics simulation. In a third training phase, the processing circuitry receives a predicted value for a property of sampled protein structures, compares the predicted value to an actual value of the property, and backpropagates the diffusion model with the difference. The diffusion model estimates a denoised protein structure corresponding to the given protein sequence in fifteen or fewer denoising steps.

"And/or" as used herein is defined as the inclusive or V, as specified by the following truth table:

| A | B | A∨B |
|---|---|---|
| True | True | True |
| True | False | True |
| False | True | True |
| False | False | False |

It will be understood that the configurations and/or approaches described herein are exemplary in nature, and that these specific embodiments or examples are not to be considered in a limiting sense, because numerous variations are possible. The specific routines or methods described herein may represent one or more of any number of processing strategies. As such, various acts illustrated and/or described may be performed in the sequence illustrated and/or described, in other sequences, in parallel, or omitted. Likewise, the order of the above-described processes may be changed.

The subject matter of the present disclosure includes all novel and non-obvious combinations and sub-combinations of the various processes, systems and configurations, and other features, functions, acts, and/or properties disclosed herein, as well as any and all equivalents thereof.

# EP 4 738 367 A1

**Claims**

1.  A computing system (10) for predicting protein structure ensembles, comprising:
    a computing device (14) including processing circuitry (18) configured to execute instructions (28) using portions of associated memory (22) to implement a protein structure ensemble prediction model (30), the processing circuitry (18) being configured to:

    in a first training phase,

    ingest a synthetic dataset of protein sequences,
    identify protein sequences in the synthetic dataset having structurally heterogeneous predictions,
    perform structure-based clustering on the identified protein sequences based on the structurally heterogeneous predictions to produce clusters of predicted protein structures,
    filter the clusters of predicted protein structures to remove disordered predicted protein structures and clusters comprising a single predicted protein structure,
    generate training pairs for a diffusion model (66) included in the protein structure ensemble prediction model (30), and
    train the diffusion model (66) on the training pairs;

    in a second training phase,

    sample a training protein sequence and a corresponding training protein structure from a molecular dynamics simulation,
    corrupt the corresponding training protein structure,
    input the training protein sequence and the corrupted version of the corresponding training protein structure into the diffusion model (66),
    receive, from the diffusion model (66), a predicted uncorrupted protein structure corresponding to the input training protein sequence, and
    compare the predicted uncorrupted protein structure from the diffusion model (66) to the corresponding training protein structure sampled from the molecular dynamics simulation; and

    in a third training phase,

    instruct the diffusion model (66) to sample a plurality of structures for a given protein sequence,
    receive, from the diffusion model (66), a predicted value for a property of a distribution of the plurality of sampled structures,
    compare the predicted value of the property from the diffusion model (66) to an actual value of the property,
    calculate a difference between the predicted value of the property and the actual value of the property, and
    backpropagate the diffusion model (66) with the calculated difference to minimize a loss function, wherein

    the diffusion model (66) is configured to estimate a denoised protein structure corresponding to the given protein sequence in fifteen or fewer denoising steps.

2.  The computing system according to claim 1, wherein
    the protein sequences having structurally heterogeneous predictions are identified via many-against-many sequence searching, and/or wherein the structure-based clustering is performed using a protein structure alignment server.

3.  The computing system according to any one of claim 1 or claim 2, wherein
    to generate the training pairs for the diffusion model, the processing circuitry is configured to:

    randomly select a predicted protein structure from a randomly selected cluster of predicted protein structures, and
    pair the randomly selected predicted protein structure with a protein sequence that corresponds to a predicted protein structure having a highest predicted local distance different test value from within the randomly selected cluster.

4.  The computing system according to any one of claims 1 to 3, wherein

    the property of the distribution of the plurality of sampled structures is one of a class, a value, and a tensor, and

optionally, wherein the property is a value of a free energy difference between different metastable states or a mean value of a distance between two amino acids in a three-dimensional structure of the protein structure or wherein the value of the free energy difference includes a value of a free energy difference between folded and unfolded states of the protein structure.

**5.** The computing system according to any one of claims 1 to 4, wherein

when the molecular dynamics simulation does not reach equilibrium within a simulation timeframe, a re-weighting procedure is performed over protein structures in the molecular dynamics simulation to approximate an equilibrium distribution, and
during the second training phase, the corresponding training protein structure is sampled from the molecular dynamics simulation with a probability according to the re-weighted protein structures.

**6.** A computerized method (400) for training a model (30) to predict protein structure ensembles utilizing processing circuitry (18) and memory (22) of one or more computing devices (14), the method comprising:

in a first training phase,

ingesting (402) a synthetic dataset of protein sequences,
identifying (404) protein sequences in the synthetic dataset having structurally heterogeneous predictions,
performing (406) structure-based clustering on the identified protein sequences based on the structurally heterogeneous predictions to produce clusters of predicted protein structures,
filtering (408) the clusters of predicted protein structures to remove disordered predicted protein structures and clusters comprising a single predicted protein structure,
generating (410) training pairs for a diffusion model (66) included in the protein structure ensemble prediction model (30), and
training (412) the diffusion model (66) on the training pairs;

in a second training phase,

sampling (414) a training protein sequence and a corresponding training protein structure from a molecular dynamics simulation,
corrupting (416) the corresponding training protein structure,
inputting (418) the training protein sequence and the corrupted version of the corresponding training protein structure into the diffusion model (66),
receiving (420), from the diffusion model (66), a predicted uncorrupted protein structure corresponding to the input training protein sequence, and
comparing (422) the predicted uncorrupted protein structure from the diffusion model (66) to the corresponding training protein structure sampled from the molecular dynamics simulation; and

in a third training phase,

instructing (424) the diffusion model (66) to sample a plurality of structures for a given protein sequence,
receiving (426), from the diffusion model (66), a predicted value for a property of a distribution of the plurality of sampled structures,
comparing (428) the predicted value of the property from the diffusion model (66) to an actual value of the property,
calculating (430) a difference between the predicted value of the property and the actual value of the property, and
backpropagating (432) the diffusion model (66) with the calculated difference to minimize a loss function, wherein

the diffusion model (66) is configured to estimate a denoised protein structure corresponding to the given protein sequence in fifteen or fewer denoising steps.

**7.** The computerized method according to claim 9, further comprising:
identifying the protein sequences having structurally heterogeneous predictions via many-against-many sequence searching, and/or performing the structure-based clustering with a protein structure alignment server.

8. The computerized method according to any one of claims 6 or claim 7, further comprising:
generating the training pairs for the diffusion model by:

randomly selecting a predicted protein structure from a randomly selected cluster of predicted protein structures, and
pairing the randomly selected predicted protein structure with a protein sequence that corresponds to a predicted protein structure having a highest predicted local distance different test value from within the randomly selected cluster.

9. The computerized method according to any one of claims 6 to 8, wherein
the property of the distribution of the plurality of sampled structures is one of a class, a value, and a tensor.

10. The computerized method according to claim 9, wherein

the property is a value of a free energy difference between different metastable states or a mean value of a distance between two amino acids in a three-dimensional structure of the protein structure, and
optionally, wherein the value of the free energy difference includes a value of a free energy difference between folded and unfolded states of the protein structure.

11. The computerized method according to any one of claims 6 to 14, further comprising:

when the molecular dynamics simulation does not reach equilibrium within a simulation timeframe, performing a re-weighting procedure over protein structures in the molecular dynamics simulation to approximate an equilibrium distribution, and
during the second training phase, sampling the corresponding training protein structure from the molecular dynamics simulation with a probability according to the re-weighted protein structures.

12. A computing system (10) for predicting protein structure ensembles, comprising:
a computing device (14) including processing circuitry (18) configured to execute instructions (28) using portions of associated memory (22) to implement a protein structure ensemble prediction model (30), the processing circuitry (18) being configured to:
in an inference phase,

receive an input protein sequence (32);
perform a search for protein sequence data (46) based on the input protein sequence (32);
identify and retrieve a subset of the protein sequence data (46) having similarity to the input protein sequence (32);
perform a multiple sequence alignment between the input protein sequence (32) and the subset of the protein sequence data (46) to produce multiple sequence alignment data (52);
encode data from the multiple sequence alignment, the encoded data (62) including single representations (62A) corresponding to the multiple sequence alignment data (52); and
input the encoded data (62) into a denoising diffusion model (66) to predict molecular properties and structural features of the input protein sequence (32).

13. The computing system according to claim 12, wherein the processing circuitry is further configured to:

perform a search for protein structure data based on the input protein sequence;
identify and retrieve candidate protein structure data for candidates having a sequence-structure relationship with the input protein sequence;
pair the input protein sequence and candidate protein structure data to produce pair data; and
encode data from the pairing of the input protein sequence and the candidate protein structure data, the encoded data including pair representations corresponding to the pair data.

14. The computing system according to claim 13, wherein

the multiple sequence alignment data and the pair data from the pairing of the input sequence and the candidate protein structure data are input to a refinement model, and
the refinement model outputs a joint latent representation as encoded data, the encoded data including the single representations corresponding to the multiple sequence alignment data and the pair representations corresponding to the pair data.

15. The computing system according to any one of claims 12 to 14, wherein the multiple sequence alignment between the input protein sequence and the subset of the protein sequence data is expressed as graph-structured data.

FIG. 1

FIG. 2A

PROTEIN SEQUENCE ENCODER MODULE 42

SEQUENCE 32

SEQUENCE DATA 46

MSA

MSA DATA 52

PAIRING

PROT STRUCT DATA 48

PAIR DATA 58

REFINEMENT MODEL (EVOFORMER) 60

ENCODED DATA 62

SINGLE REPR 62A

TO FIG. 2B →

PAIR REPR 62B

EP 4 738 367 A1

FIG. 2B

PROTEIN STRUCTURE DECODER MODULE 64

DENOISING DIFFUSION MODEL 66

$x_T$ • • • $x_{t-dt}$ $x_t$ • • • $x_0$

EQUILIBRIUM DISTRIBUTION 78

FROM FIG. 2A

DIF TIMESTEP $t$

SINGLE REPR 62A

PAIR REPR 62B

BACKBONE FRAMES $x_t$

SCORE MODEL 68

NODE FEATURE 70

IPA

NODE FEATURE 70

MLP

SCORE 72

NOISE 74

EP 4 738 367 A1

FIG. 3A — PRE-TRAINING

- SYNTHETIC DATASET
- MMseq + Filtering
- 1.4 M SEQ. CLUSTERS
- PSAS + filtering
- 50 K SEQ. CLUSTERS WITH DIVERSE STRUCTURES
- multi-struct. Augmentation
- AUGMENTED CLUSTER AFDB
- Diffusion Model Training
- PRE-TRAINED MODEL

Pretrained Distribution

FIG. 3B — FINE-TUNING I

- > 200 ms MD SIMULATIONS
- Re-weighting
- REWEIGHTED MD
- TRUE UNCORRUPTED PROTEIN STRUCTURE
- Corrupting
- PROT. SEQ. — CORRUPTED STRUCT.
- PREDICTED UNCORRUPTED PROTEIN STRUCTURE
- Comparing
- INTERMEDIATE MODEL

MD Distribution

FIG. 3C — FINE-TUNING II

- 500 K EXP. PROTEIN STABILITIES
- PROPERTY-PREDICTION FINE-TUNING
  - $X_T$ ... $X_{T-k\,dt}$ — predict — classify — FOLDED / UNFOLDED
  - backprop
  - $X_0$
  - PROPERTY PREDICTION LOSS
- EXP. DATA $\Delta G = 3.5\,kcal/mol$
- FINE-TUNED MODEL

Equilibrium Distribution

FIG. 3A    FIG. 3B    FIG. 3C

EP 4 738 367 A1

400

IN A FIRST TRAINING PHASE:

INGESTING A SYNTHETIC DATASET OF PROTEIN SEQUENCES  402

↓

IDENTIFYING PROTEIN SEQUENCES HAVING STRUCTURALLY HETEROGENEOUS PREDICTIONS 404

↓

PERFORMING STRUCTURE-BASED CLUSTERING BASED ON THE STRUCTURALLY HETEROGENEOUS PREDICTIONS 406

↓

FILTERING THE CLUSTERED PROTEIN SEQUENCES 408

↓

GENERATING TRAINING PAIRS FOR A DIFFUSION MODEL 410

↓

TRAINING THE DIFFUSION MODEL ON THE TRAINING PAIRS 412

↓

TO FIG. 4B

FIG. 4A

400

FROM FIG. 4A

IN A SECOND TRAINING PHASE (I.E., FIRST FINE-TUNING STEP):

SAMPLING A TRAINING PROTEIN SEQUENCE AND A CORRESPONDING TRAINING PROTEIN STRUCTURE FROM A MOLECULAR DYNAMICS SIMULATION 414

CORRUPTING THE CORRESPONDING TRAINING PROTEIN STRUCTURE 416

INPUTTING THE TRAINING PROTEIN SEQUENCE AND THE CORRUPTED VERSION OF THE CORRESPONDING TRAINING PROTEIN STRUCTURE INTO THE DIFFUSION MODEL 418

RECEIVING A PREDICTED UNCORRUPTED PROTEIN STRUCTURE FROM THE DIFFUSION MODEL 420

COMPARING THE PREDICTED UNCORRUPTED PROTEIN STRUCTURE TO AN UNCORRUPTED CORRESPONDING PROTEIN STRUCTURE 422

TO FIG. 4C

FIG. 4B

400

FROM FIG. 4B

IN A THIRD TRAINING PHASE (I.E., SECOND FINE-TUNING STEP):

INSTRUCTING THE DIFFUSION MODEL TO SAMPLE A PLURALITY OF STRUCTURES FOR A GIVEN PROTEIN SEQUENCE 424

RECEIVING A PREDICTED VALUE FOR A PROPERTY OF A DISTRIBUTION OF THE PLURALITY OF SAMPLED STRUCTURES 426

COMPARING THE PREDICTED VALUE OF THE PROPERTY FROM THE DIFFUSION MODEL TO AN ACTUAL VALUE OF THE PROPERTY 428

CALCULATING A DIFFERENCE BETWEEN THE PREDICTED VALUE OF THE PROPERTY AND THE ACTUAL VALUE OF THE PROPERTY 430

BACKPROPAGATING THE DIFFUSION MODEL WITH THE CALCULATED DIFFERENCE TO MINIMIZE A LOSS FUNCTION 432

FIG. 4C

COMPUTING SYSTEM 500

LOGIC PROCESSOR 502

VOLATILE MEMORY 504

NON-VOLATILE STORAGE DEVICE 506

DISPLAY SUBSYSTEM 508

INPUT SUBSYSTEM 510

COMMUNICATION SUBSYSTEM 512

FIG. 5

Europäisches
Patentamt

European
Patent Office

Office européen
des brevets

## EUROPEAN SEARCH REPORT

**Application Number**

EP 25 21 1210

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2024/187031 A2 (IAMBIC THERAPEUTICS INC [US]; CALIFORNIA INST OF TECHN [US] ET AL.) 12 September 2024 (2024-09-12) | 12-15 | INV.<br>G16B15/00<br>G16B15/20 |
| A | * paragraphs [0001] - [0023] *<br>* paragraphs [0085] - [0475] *<br>* paragraphs [0565] - [0575] *<br>* paragraphs [0595] - [0598] *<br>* claims 1-37 *<br>* figure 1A * | 1-11 | G16B40/20 |
| X | US 2024/161864 A1 (INGRAHAM JOHN [US]) 16 May 2024 (2024-05-16) | 12-15 | |
| A | * paragraph [0007] - paragraph [0020] *<br>* paragraph [0070] - paragraph [0075] *<br>* paragraph [0079] - paragraph [0080] *<br>* paragraph [0086] - paragraph [0088] *<br>* paragraph [0093] - paragraph [0112] *<br>* paragraph [0122] - paragraph [0127] *<br>* paragraph [0205] - paragraph [0218] * | 1-11 | |
| X | ZHUORAN QIAO ET AL: "Dynamic-Backbone Protein-Ligand Structure Prediction with Multiscale Generative Diffusion Models", ARXIV.ORG, CORNELL UNIVERSITY LIBRARY, 201 OLIN LIBRARY CORNELL UNIVERSITY ITHACA, NY 14853, 30 September 2022 (2022-09-30), XP091330337, | 12-15 | **TECHNICAL FIELDS SEARCHED (IPC)**<br><br>G16B |
| A | * the whole document *<br>* in particular *<br>* abstract *<br>* Methods * | 1-11 | |
| A | WO 2024/158466 A2 (UNIV WASHINGTON [US]) 2 August 2024 (2024-08-02)<br>* paragraph [0044] - paragraph [0229] * | 1-15 | |

-----

-/--

The present search report has been drawn up for all claims

| Place of search | Date of completion of the search | Examiner |
|---|---|---|
| Munich | 27 February 2026 | Martínez Cebollada |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
........................................................................................
& : member of the same patent family, corresponding document

EPO FORM 1503 03.82 (P04C01)

**EUROPEAN SEARCH REPORT**

Application Number

EP 25 21 1210

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | BOZHEN HU ET AL: "Advances of Deep Learning in Protein Science: A Comprehensive Survey", ARXIV.ORG, CORNELL UNIVERSITY LIBRARY, 201 OLIN LIBRARY CORNELL UNIVERSITY ITHACA, NY 14853, 8 March 2024 (2024-03-08), XP091695600, * the whole document * ----- | 1-15 | |

TECHNICAL FIELDS
SEARCHED       (IPC)

The present search report has been drawn up for all claims

| Place of search | Date of completion of the search | Examiner |
|---|---|---|
| Munich | 27 February 2026 | Martínez Cebollada |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 25 21 1210

This annex lists the patent family members relating to the patent documents cited in the above-mentioned European search report.
The members are as contained in the European Patent Office EDP file on
The European Patent Office is in no way liable for these particulars which are merely given for the purpose of information.

27-02-2026

| Patent document cited in search report | Publication date | Patent family member(s) | | Publication date |
|---|---|---|---|---|
| WO 2024187031 A2 | 12-09-2024 | EP | 4677594 A2 | 14-01-2026 |
| | | WO | 2024187031 A2 | 12-09-2024 |
| US 2024161864 A1 | 16-05-2024 | EP | 4616403 A1 | 17-09-2025 |
| | | US | 2024161864 A1 | 16-05-2024 |
| | | US | 2025218530 A1 | 03-07-2025 |
| | | US | 20260038629 A1 | 05-02-2026 |
| | | WO | 2024102413 A1 | 16-05-2024 |
| WO 2024158466 A2 | 02-08-2024 | NONE | | |

EPO FORM P0459

For more details about this annex : see Official Journal of the European Patent Office, No. 12/82